**Europäisches Patentamt**

**European Patent Office**

**Office européen des brevets**

(19)

(11) Numéro de publication: **0 136 212**
A1

# (12) DEMANDE DE BREVET EUROPEEN

(21) Numéro de dépôt: **84401631.1**

(22) Date de dépôt: **03.08.84**

(51) Int. Cl.⁴: **A 61 B 5/02**

---

(30) Priorité: **03.08.83 FR 8312822**

(43) Date de publication de la demande: **03.04.85**
**Bulletin 85/14**

(84) Etats contractants désignés: **AT BE CH DE GB IT LI LU
NL SE**

(71) Demandeur: **Berger, Henri, 90, Boulevard de
Latour-Maubourg, F-75007 Paris (FR)**

(72) Inventeur: **Berger, Henri, 90, Boulevard de
Latour-Maubourg, F-75007 Paris (FR)**
Inventeur: **Lapeyre, Didler, Chaignes, F-27120 Pacy sur
Eure (FR)**

(74) Mandataire: **Gorree, Jean-Michel et al, Cabinet
Plasseraud 84, rue d'Amsterdam, F-75009 Paris (FR)**

---

(54) **Appareil et procédé pour mesure la tension artérielle suivant une méthode indirecte.**

(57) L'invention vise à mesurer par voie externe la pression artérielle d'un patient en utilisant au moins un capteur de force (19) destiné à être tenu en appui dans la gouttière du pouls avec une force constante inférieure à celle créée par la pression diastolique du flux sanguin de l'artère radiale, en détectant (21, 25, 27, 30) les maxima et les minima des signaux de pression, en calculant (29, 32, 33) la moyenne des rapaports R de ces maxima et minima, en augmentant la force d'appui jusqu'à perturber le flux sanguin (pression diastolique $P_D$), en calculant la pression systolique $P_S = P_D \times R_m$ et en affichant (17) les valeurs $P_S$ et $P_D$.

0136212

<u>Appareil et procédé pour mesurer la tension artérielle</u>
<u>suivant une méthode indirecte</u>

La présente invention concerne un appareil et un procédé de mesure de la tension artérielle suivant une méthode indirecte, non sanglante (c'est-à-dire, par exemple, sans introduction de cathéter). Le mode opératoire classique de détermination de la tension artérielle, dit de RIVA-ROCCI, consiste à bloquer une partie du système circulatoire du malade en exerçant sur son bras une pression supérieure à la pression systolique afin d'empêcher le sang de s'écouler vers l'avant-bras, puis à relâcher très lentement cette pression en relevant sur un manomètre les deux valeurs pour lesquelles les bruits de KOROTKOV apparaissent, s'assourdissent, puis disparaissent.

Lorsque les bruits apparaissent, on note la pression de crête ou pression systolique ; lorsque les bruits disparaissent, on note la pression minimale ou pression diastolique.

Un premier inconvénient de cette méthode est qu'elle nécessite, en plus d'un stéthoscope, un brassard gonflable encombrant dont les dimensions doivent être adaptées à la grosseur du bras.

Un deuxième inconvénient de cette méthode est que la relation entre la pression diastolique, qui est pourtant la plus représentative de l'état circulatoire du malade, et l'assourdissement ou la disparition totale des bruits de KOROTKOV est très controversée dans le milieu médical.

Un troisième inconvénient de cette méthode est que les bruits parasites émis dans la pièce ou provoqués par un mouvement du patient rendent l'écoute des bruits de KOROTKOV très délicate et nécessitent un opérateur expérimenté.

De nombreux perfectionnements ont été proposés, consistant essentiellement à rendre automatiques le gonflage du brassard, la mesure de la pression, ou l'analyse des bruits, comme par exemple le brevet FR 75 05046 (publié sous le n° 2.260.975), mais ils conduisent généralement à un appareillage lourd, encombrant, et aléatoire.

L'invention a essentiellement pour but de supprimer tous ces inconvénients dans toute la mesure du possible et de permettre à tout un chacun de mesurer sur lui-même aussi facilement que sur quelqu'un d'autre les pressions diastolique et systolique, et accessoirement la pression moyenne et le rythme cardiaque, avec une fiabilité réservée jusqu'ici aux spécialistes expérimentés, et cela sans jamais interrompre le flux sanguin, contrairement à tous les appareils et procédés existants qui interrompent le flux sanguin totalement, puis partiellement pendant de nombreuses secondes.

A ces fins, selon un premier de ses aspects qui est relatif à un appareil pour mesurer la tension artérielle, l'invention prévoit que ledit appareil comprend :

- au moins un capteur de force destiné, en fonctionnement, à être tenu en appui dans la gouttière du pouls avec une force sensiblement constante inférieure à celle créée par la pression diastolique du flux sanguin de l'artère radiale,

- des moyens de détection connectés au susdit capteur de force et agencés pour détecter les maxima et les minima des signaux de sortie du capteur de force,

- des premiers moyens de mémorisation connectés auxdits moyens de détection et agencés pour mémoriser lesdits maxima et lesdits minima,

- des premiers moyens de calcul connectés auxdits premiers moyens de mémorisation et agencés pour calculer respectivement la somme des maxima du signal de pression et la somme des minima du signal de pression,

- des deuxièmes moyens de calcul connectés aux premiers moyens de calcul et agencés pour calculer la valeur moyenne $R_m$ du rapport de la somme des maxima du signal de pression à la somme des minima du signal de pression,
- des deuxièmes moyens de mémorisation connectés au capteur de force et agencés pour mémoriser la valeur de la force d'appui correspondant à l'amorce de la perturbation du flux sanguin, cette force étant considérée comme la pression diastolique $P_D$,
- des troisièmes moyens de calcul connectés aux deuxièmes moyens de mémorisation et aux deuxièmes moyens de calcul et agencés pour calculer la valeur $P_S$ du produit de la susdite valeur $P_D$ par la susdite valeur $R_m$,
- et des moyens d'affichage connectés auxdits troisièmes moyens de calcul et aux deuxièmes moyens de mémorisation et agencés pour afficher, simultanément et dans l'ordre, les valeurs $P_S$ et $P_D$.

Il peut être avantageux d'agencer l'appareil de l'invention de manière à lui faire mesurer, en outre, la pression artérielle moyenne dont la connaissance peut être souhaitée en complément des pressions systolique et diastolique, par exemple pour déceler des problèmes cardiaques que la connaissance des seules pressions systolique et diastolique ne permet pas de déterminer. A cette fin, l'appareil de l'invention comprend en outre :

- des quatrièmes moyens de calcul connectés aux moyens de détection et agencés pour calculer la valeur intégrale du signal au cours d'un cycle,
- des cinquièmes moyens de calcul connectés aux quatrièmes moyens de calcul et aux moyens de détection des minima et agencés pour effectuer le quotient M de la susdite valeur intégrale par la durée du cycle (temps séparant deux maxima consécutifs) et par le minimum du cycle considéré,
- des troisièmes moyens de mémorisation connectés aux cinquièmes moyens de calcul et agencés pour mémoriser les rapports M calculés par lesdits cinquièmes moyens de calcul,

- des sixièmes moyens de calcul connectés aux troisièmes moyens de mémorisation et agencés pour calculer la valeur moyenne des rapports M mémorisés dans lesdits troisièmes moyens de mémorisation,
- des septièmes moyens de calcul connectés aux deuxièmes moyens de mémorisation et aux sixièmes moyens de calcul et agencés pour calculer le produit, considéré comme étant la valeur de la pression moyenne $P_M$, de la valeur de la pression diastolique $P_D$ par la valeur de la moyenne des rapports M, lesdits septièmes moyens de calcul ayant leur sortie connectée auxdits moyens d'affichage pour l'affichage de la pression moyenne $P_M$.

Il peut également être avantageux d'agencer l'appareil de l'invention de manière à lui faire mesurer en outre le rythme cardiaque, afin, par exemple, de déceler une éventuelle arythmie. A cette fin, l'appareil de l'invention comprend en outre :

- des huitièmes moyens de calcul connectés aux quatrièmes moyens de calcul et agencés pour calculer l'inverse de la valeur de la période T fournie par les quatrièmes moyens de calcul et la multiplier par 60, ces huitièmes moyens de calcul ayant leur sortie connectée aux moyens d'affichage en vue de l'affichage, au cours de chaque cycle cardiaque, du rythme cardiaque.

Pour faciliter la détection de la pression diastolique, il est souhaitable que l'appareil comporte en outre ou qu'il lui soit associé des moyens d'analyse de la forme de la variation instantanée de la pression artérielle, ces moyens d'analyse ayant leur sortie connectée à une entrée de commande des seconds moyens de mémorisation de manière à commander la mise en mémoire de la force F exercée par le capteur de force.

Dans ce cas, un mode de réalisation préféré de l'appareil de l'invention se caractérise en ce que les moyens d'analyse comprennent au moins un second capteur de force,

0136212

5

les deux capteurs étant destinés, dans la position de fonctionnement de l'appareil, à être disposés l'un à la suite de l'autre dans la gouttière du pouls le long de l'artère radiale, et en ce que sont en outre prévus :

- deux groupes de moyens de détection connectés respectivement aux deux capteurs, et
- des moyens comparateurs dont deux entrées sont connectées respectivement aux deux groupes de moyens de détection et dont la sortie est connectée à des moyens de signalisation du positionnement de l'appareil.

Le recours à deux capteurs de pression, destinés à être disposés l'un à la suite de l'autre le long de l'artère radiale tout en étant connectés en parallèle dans le circuit de traitement d'information (avec unité soustractrice et unité sommatrice pour comparer les signaux de sortie de ces deux capteurs), offre en outre l'avantage supplémentaire important que, avec un circuit de signalisation approprié. l'appareil ne sera en état de fournir des indications valables que si les deux capteurs sont exactement situés le long de l'artère radiale et s'ils exercent sur celle-ci une même force ; dans le cas contraire. l'affichage d'information peut par exemple être bloqué sous la commande du circuit de signalisation, et l'utilisateur est ainsi informé que l'appareil est mal positionné.

Un autre avantage supplémentaire importantoffert par le recours à deux capteurs de force disposés l'un à la suite de l'autre le long de l'artère radiale est que l'appareil objet de la présente invention peut. lorsqu'il est appuyé sur ladite artère avec une force supérieure à celle exercée par la pression systolique du flux sanguin et que cette force d'appui est ensuite progressivement diminuée jusqu'à être annulée. analyser l'amplitude des oscillations pour déterminer la pression systolique et la pression diastolique du flux sanguin en utilisant la méthode bien connue de l'oscillométrie.

0136212

6

Afin de faciliter son utilisation l'appareil peut être muni d'un des dispositifs de préhension et de suspension bien connus des hommes de l'art et non représentés ici permettant aux deux capteurs d'être appuyés avec une même force progressivement croissante ou progressivement décroissante alors que l utilisateur de l'appareil exerce une force constante ou nulle.

L'appareil de l invention peut par conséquent être utilisé par toute personne. même non expérimentée.

Selon un second des aspects de l'invention qui est relatif à un procédé pour mesurer la tension artérielle, l'invention prévoit que ledit procédé comprend les étapes suivantes :

- on appuie au moins un capteur de force dans la gouttière du pouls avec une force sensiblement constante inférieure à celle créée par la pression diastolique du flux sanguin de l'artère radiale,
- on détecte les maxima et les minima des signaux fournis par le capteur et représentatifs de la forme de la pression artérielle, pendant une durée de temps correspondant à plusieurs cycles cardiaques,

- on détermine, pour chaque cycle cardiaque, le rapport R du maximum du signal de pression au minimum du signal de pression mesurés au cours de chaque cycle cardiaque,

- on augmente progressivement la force d'appui du capteur jusqu'à perturber le flux sanguin, on détecte la déformation du signal de pression et on considère la force d'appui correspondant à l'amorce de la perturbation comme étant la pression diastolique $P_D$,

- on détermine, à la fin de la susdite durée de temps, la moyenne des rapports R précédemment calculés,

- on détermine le produit de la valeur de la pression diastolique $P_D$ par la moyenne des rapports R, ce produit étant considéré comme étant la valeur de la pression systolique $P_S$, et

- on affiche les valeurs $P_S$ et $P_D$.

Si l'on souhaite en outre déterminer la pression artérielle moyenne, on prévoit les étapes suivantes :

- on détermine, pour chaque cycle cardiaque, le rapport M de la pression moyenne, intégrale du signal fourni par le capteur pour ledit cycle divisé par la durée du cycle, au minimum mesuré au cours du cycle considéré,

- on détermine, à la fin de la susdite durée de temps, la valeur moyenne des rapports M,

- on détermine alors le produit de la valeur de la pression diastolique $P_D$ par la moyenne des rapports M, ce produit étant considéré comme étant la valeur de la pression artérielle moyenne $P_M$, et

- on affiche la valeur $P_M$.

Enfin, si l'on souhaite encore en outre déterminer le rythme cardiaque, on prévoit les étapes suivantes :

- on détecte la période de chaque cycle cardiaque,

- on détermine l'inverse de cette période du cycle cardiaque et on multiplie par 60 cette valeur inverse, et

- on affiche la valeur du rythme cardiaque.

8

Dans un aspect préféré du procédé de l'invention, on utilise au moins deux capteurs de force disposés l'un à la suite de l'autre dans la gouttière du pouls le long de l'artère radiale, et on compare les signaux fournis par ces deux capteurs pour déterminer que la force d'appui est inférieure ou respectivement au moins égale à la force créée par la pression diastolique du flux sanguin de l'artère radiale selon que les signaux fournis sont identiques ou respectivement différents.

D'une façon générale, on notera que l'invention, contrairement à tous les appareils et procédés connus à ce jour y compris celui décrit dans le brevet FR 70 07315 (numéro de publication 2.063.833), est la seule qui procède par analyse de la forme du flux artériel pour des forces appliquées à la paroi extérieure d'une artère qui sont inférieures à la force exercée sur la paroi intérieure de ladite artère par la pression diastolique du flux artériel. L'invention est basée sur la mesure de la force exercée sur les membranes de deux dynamomètres, d'une part, par l'utilisateur de l'appareil appuyant celui-ci sur l'artère radiale dans la gouttière du pouls, d'autre part, par l'onde pulsatile du flux artériel à travers la paroi de ladite artère; plus précisément, elle est basée sur l'analyse de la forme des deux mesures de ces forces en deux points voisins comme le font les deux doigts du praticien palpant l'artère radiale dans la gouttière du pouls, mais cela statistiquement à partir des valeurs échantillonnées de la mesure fournie par les dynamomètres.

L'invention sera mieux comprise à la lecture de la description détaillée qui suit de la structure et de l'utilisation d'un mode de réalisation préféré d'un appareil agencé conformément à l'invention, lequel mode de réalisation préféré n'est donné qu'à titre illustratif sans aucun caractère limitatif. Dans cette description, on se réfère aux dessins 1 à 6 ci-annexés sur lesquels :

- la figure 1 est un graphique donnant un exemple d'évolution de la pression artérielle en fonction du temps ;

- la figure 2 est un graphique donnant des exemples des tensions fournies par le premier des deux dynamomètres en fonction du temps pour trois valeurs de la force exercée par l'utilisateur de l'appareil ;
- la figure 3 est une vue de profil d'un mode de réalisation préféré de l'appareil suivant l'invention ;
- la figure 4 est une vue de face de l'afficheur de l'appareil (vue suivant la flèche IV sur la figure 3) ;
- la figure 5 donne une vue de face de la partie de l'appareil que l'utilisateur appuie dans la gouttière du pouls (vue suivant la flèche V sur la figure 3) ;
- la figure 6 est un schéma-bloc illustrant la structure de l'appareil des figures 3 à 5; et
- la figure 7 est un schéma-bloc illustrant une autre structure d'un appareil conforme à l'invention.

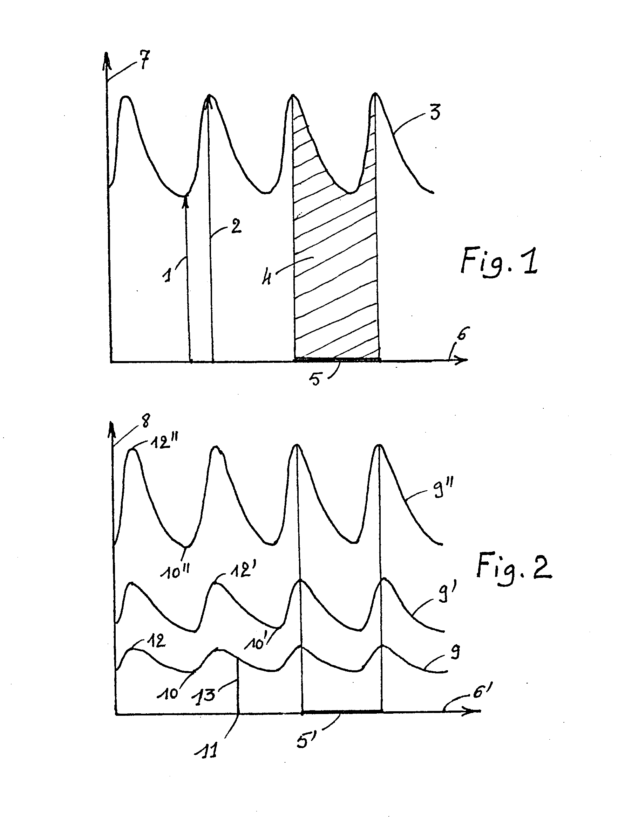

La figure 1 représente l'évolution. en foction du temps courant suivant l'axe 6. d'un exemple de quatre cycles de pression artérielle 3 portée suivant l'axe 7.

L'amplitude 1 représente la pression minimum ou pression diastolique $P_D$. l'amplitude 2 représente la pression maximum ou pression systolique $P_S$. Le segment 5 représente la grandeur T d'une période du cycle cardiaque. La pression moyenne $P_M$ est obtenue en divisant l aire 4 par le segment 5. Dans la suite de cette description. on appellera M le rapport $P_M/P_D$.

La figure 2 représente l'évolution. en fonction du temps courant suivant l'axe 6'. d'un exemple des tensions (exprimées en volts) portées suivant l'axe 8 fournies par l'un des dynamomètres utilisés pour détecter la pression artérielle .La tension 9 est obtenue pour une force d'appui très faible. la tension 9' est obtenue pour une force d appui double de la précédente. et la tension 9" est obtenue pour une force d'appui quadruple de la première. Ces tensions sont pratiquement proportionnelles à la force d'appui exercée sur l'artère.

Les tensions fournies par le second dynamomètre ne sont pas représentées. mais elles sont exactement identiques aux tensions 9. 9' et 9" à un décalage près $n\tau$ suivant l'axe 6' de quelques périodes d'échantillonnage $\tau$ dont il est tenu compte par l'électronique.

On remarquera sur cette figure 2 que le segment 5' est identique au segment 5 de la figure 1 et qu'il a une grandeur égale à T.

On remarquera également l'échantillonnage 13 de la tension 9 effectué à l'instant 11.

On remarquera surtout en comparant les figures 1 et 2. ce qui est le fondement de la présente invention. que le rapport des amplitudes 2 et 1. c'est-à-dire $P_S/P_D$ de la figure 1 et qui sera désigné par R dans la suite de cette description. est pratiquement égal aux rapports des amplitudes 12/10. 12'/10' et 12"/10" des maxima aux minima des tensions 9. 9' et 9".

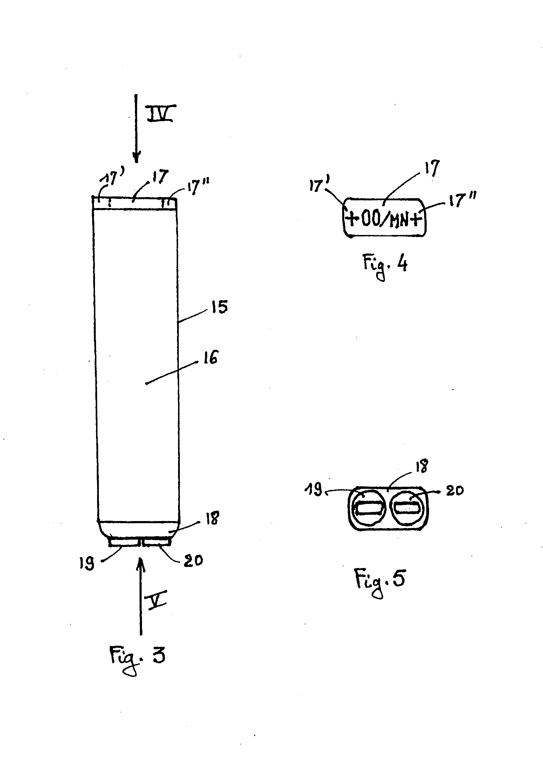

La figure 3 représente une vue de profil d'un mode de réalisation préféré de l'appareil suivant l'invention qui a la forme d'un parallélépidède 15 aux arêtes arrondies qui peut avoir par exemple environ 8 cm de long et une section de 1 cm sur 2 cm. L'extrémité 18 est légèrement bombée et comporte. alignées l'une dans le prolongement de l'autre à quelques millimètres de distance. deux touches 19 et 20 de 1 cm de diamètre environ ne laissant apparaître les membranes de deux dynamomètres qu'au travers d'une ouverture rectangulaire de 0.4 cm sur 0.8 cm environ.

L'autre extrémité est constituée par un afficheur 17 d'au moins cinq caractères alphanumériques. une afficheur 17' et un afficheur 17" d'au moins 1 caractère chacun.

Le corps 16 de l'appareil contient une pile électrique. un interrupteur mettant 1 appareil sous tension lorsqu il est serré entre le pouce et l index de l'utilisateur. et l'électronique nécessaire au fonctionnement

La figure 4 montre l'afficheur 17. et les afficheurs 17' et 17" vus de face. L'affichage +00/MN+ montré ici est celui qu'il fournit à la mise sous tension de l'appareil avant que celui-ci ne soit appuyé sur l'artère radiale.

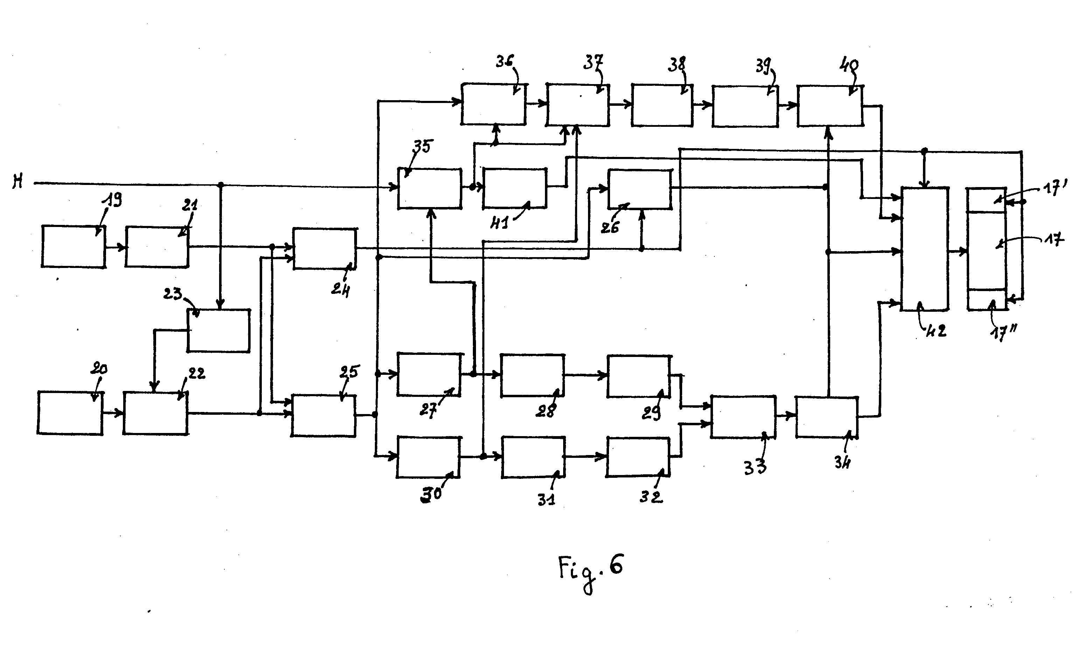

La figure 5 représente l'appareil vu de dessous d'évolution de la pression artérielle en fonction du temps ;

- la figure 2 est un graphique donnant des exemples des tensions fournies par le premier des deux dynamomètres en fonction du temps pour trois valeurs de la force exercée par l'utilisateur de l'appareil ;

- la figure 3 est une vue de profil d'un mode de réalisation préféré de l'appareil suivant l'invention ;

- la figure 4 est une vue de face de l'afficheur de l'appareil (vue suivant la flèche IV sur la figure 3) ;

- la figure 5 donne une vue de face de la partie de l'appareil que l'utilisateur appuie dans la gouttière du pouls (vue suivant la flèche V sur la figure 3) ;

- la figure 6 est un schéma-bloc illustrant la structure de l'appareil des figures 3 à 5; et

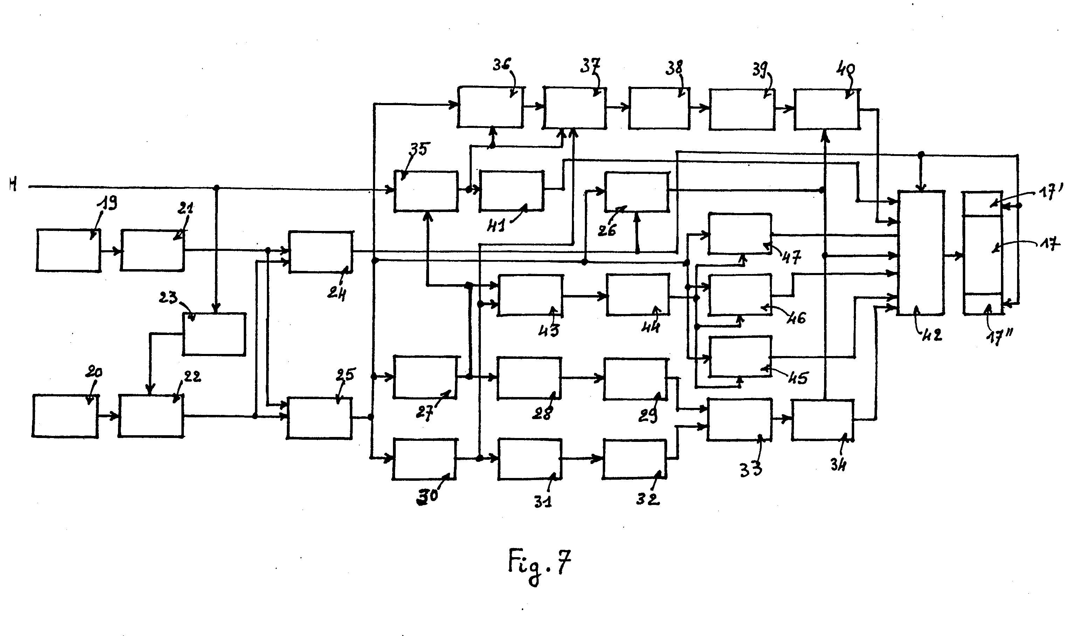

- la figure 7 est un schéma-bloc illustrant une autre structure d'un appareil conforme à l'invention.

La figure 1 représente l'évolution. en foction du temps courant suivant l'axe 6. d'un exemple de quatre cycles de pression artérielle 3 portée suivant l'axe 7.

L'amplitude 1 représente la pression minimum ou pression diastolique $P_D$. l'amplitude 2 représente la pression maximum ou pression systolique $P_S$. Le segment 5 représente la grandeur T d'une période du cycle cardiaque. La pression moyenne $P_M$ est obtenue en divisant l'aire 4 par le segment 5. Dans la suite de cette description. on appellera M le rapport $P_M/P_D$.

La figure 2 représente l'évolution. en fonction du temps courant suivant l'axe 6'. d'un exemple des tensions (exprimées en volts) portées suivant l'axe 8 fournies par l'un des dynamomètres utilisés pour détecter la pression artérielle.La tension 9 est obtenue pour une force d'appui très faible. la tension 9' est obtenue pour une force d'appui double de la précédente. et la tension 9" est obtenue pour une force d'appui quadruple de la première. Ces tensions sont pratiquement proportionnelles à la force d'appui exercée sur l'artère.

Les tensions fournies par le second dynamomètre ne sont pas représentées. mais elles sont exactement identiques aux tensions 9. 9' et 9" à un décalage près $n\tau$ suivant l'axe 6' de quelques périodes d'échantillonnage $\tau$ dont il est tenu compte par l'électronique.

On remarquera sur cette figure 2 que le segment 5' est identique au segment 5 de la figure 1 et qu'il a une grandeur égale à T.

On remarquera également l'échantillonnage 13 de la tension 9 effectué à l'instant 11.

On remarquera surtout en comparant les figures 1 et 2. ce qui est le fondement de la présente invention. que le rapport des amplitudes 2 et 1. c'est-à-dire $P_S/P_D$ de la figure 1 et qui sera désigné par R dans la suite de cette description. est pratiquement égal aux rapports des amplitudes 12/10. 12'/10' et 12"/10" des maxima aux minima des tensions 9. 9' et 9".

La figure 3 représente une vue de profil d'un mode de réalisation préféré de l'appareil suivant l'invention qui a la forme d'un parallélépidède 15 aux arêtes arrondies qui peut avoir par exemple environ 8 cm de long et une section de 1 cm sur 2 cm. L'extrémité 18 est légèrement bombée et comporte. alignées l'une dans le prolongement de l'autre à quelques millimètres de distance. deux touches 19 et 20 de 1 cm de diamètre environ ne laissant apparaître les membranes de deux dynamomètres qu'au travers d'une ouverture rectangulaire de 0.4 cm sur 0.8 cm environ.

L'autre extrémité est constiuée par un afficheur 17 d'au moins cinq caractères alphanumériques. une afficheur 17' et un afficheur 17" d'au moins 1 caractère chacun.

Le corps 16 de l'appareil contient une pile électrique. un interrupteur mettant l appareil sous tension lorsqu il est serré entre le pouce et l index de l utilisateur. et l électronique nécessaire au fonctionnement

La figure 4 montre l afficheur 17. et les afficheurs 17' et 17" vus de face. L'affichage +00/MN+ montré ici est celui qu'il fournit à la mise sous tension de l'appareil avant que celui-ci ne soit appuyé sur l'artère radiale.

La figure 5 représente l'appareil vu de dessous montrant le corps 18 contenant les dynamomètres et leurs deux touches 19 et 20.

La figure 6 montre un exemple, sous forme de schéma-bloc, de la structure générale d'un mode de réalisation préféré de l'appareil de l'invention et de la suite des opérations effectuées sur les tensions V1 et V2 fournies par les dynamomètres dans l'hypothèse où ces derniers donnent une tension analogique continue, sous le contrôle de l'horloge H, avant de parvenir à l'afficheur 17.

A l'instant t, grâce au bloc 21, la tension V1 du premier dynamomètre muni de la touche 19 est échantillonnée et numérisée, et envoyée à une entrée du comparateur 24 et à une entrée de l'additionneur 25.

A l'instant $t + n\tau$, $\tau$ étant la durée séparant deux échantillonnages consécutifs et le bloc 23 créant le décalage $n\tau$ égal à la durée du parcours du flux artériel entre les touches 19 et 20, la tension V2 du second dynamomètre muni de la touche 20 est, grâce au bloc 22, échantillonnée et numérisée, et envoyée à l'autre entrée du comparateur 24 et à l'autre entrée de l'additionneur 25.

La sortie du bloc 24 représente le test de vraisemblance qui contrôle, d'une part, la mémorisation de la force F dans le bloc 26 dont la sortie est la pression diastolique $P_D$, d'autre part, la logique de commande 42 de l'afficheur 17 et. d'autre part. les afficheurs 17' et 17".

La sortie du bloc 25 représente, à un digit près, la moyenne de V1 et de V2, c'est-à-dire Vm.

Le bloc 27 effectue la recherche des maxima de Vm qui sont mis en mémoire dans le bloc 28, et totalisés dans le bloc 29 qui effectue l'opération $\sum_{1}^{N} Vmax$ dont le résultat est envoyé au diviseur 33.

Le bloc 30 effectue, lui, la recherche des minima de Vm qui sont mis en mémoire dans le bloc 31, et totalisés dans le bloc 32 qui effectue l'opération $\sum_{1}^{N} Vmin$ dont le

résultat est lui aussi envoyé au diviseur 33.

Ce bloc 33 effectue la division de $\sum_1^N$ Vmax par $\sum_1^N$ Vmin dont le résultat, Rmoyen, est envoyé au multiplicateur 34. Le bloc 35 accumule les incréments de l'horloge H sous le contrôle des Vmax repérés par le bloc 27 ; il fournit donc la période T qui est, grâce au bloc 41, inversée et multipliée par 60 pour donner le rythme cardiaque XX/MN qui est envoyé à la logique de commande 42 de l'afficheur 17.

Le bloc 36 totalise pendant toute une période T les valeurs de Vm, ce qui représente la valeur de l'aire hachurée 4 de la figure 1.

La valeur de l'aire hachurée 4 est divisée dans le bloc 37 par la valeur du Vmin de la période considérée et par la période T elle-même, et le résultat, qui est le rapport M, est mis en mémoire dans le bloc 38.

Le bloc 39 calcule le coefficient Mmoyen en effectuant l'opération $\frac{1}{N}\sum_1^N M$ et le bloc 40 effectue la multiplication de Mmoyen par la sortie du bloc 26 qui est la pression diastolique $P_D$, ce qui donne la pression moyenne $P_M$ qui est envoyée à la logique de commande 42 de l'afficheur 17.

Le bloc 34 qui effectue la multiplication de Rmoyen venant du bloc 33 par la pression diastolique $P_D$ fournit la pression systolique $P_S$ à la logique de commande 42.

Finalement, la logique de commande 42, sous le contrôle du test de vraisemblance venant du bloc 24, envoie à l'afficheur 17 soit le rythme cardiaque, soit $P_S$ et $P_D$, soit la pression moyenne $P_M$.

Le fonctionnement de l'appareil objet de l'invention sera aisément compris par la description qui suit de son utilisation.

Dès que l'appareil est mis sous tension et alors qu'aucune force n'est exercée sur les touches de mesure 19 et 20, l'afficheur indique+OO/MN+(voir figure 4).

En effet, cinq opérations sont effectuées en permanence sur les signaux fournis par les dynamomètres :

1°) un test de vraisemblance qui consiste à vérifier que les deux mesures fournies par les deux dynamomètres respectivement sont bien identiques avec une certaine tolérance prédéterminée, montrant ainsi que les deux dynamomètres exercent une même force sur l'artère et subissent une même modulation de cette force par le flux artériel ;

2°) la détection et le classement par ordre d'amplitude, puis la mise en mémoire dans une table de chaque maximum et de chaque minimum de ces mesures ;

3°) la mesure du temps qui sépare deux maxima consécutifs, c'est-à-dire la période du rythme cardiaque ;

4°) le calcul et la mise en mémoire du rapport R de chaque maximum au minimum de la période considérée ;

5°) le calcul du rapport M de la pression moyenne $P_M$, somme des échantillonnages effectués pendant la période considérée divisée par T, au minimum de ladite période.

Lorsque l'appareil est mis sous tension mais n'est pas appuyé sur l'artère radiale, les signaux des dynamomètres sont nuls et il n'y a ni maximum ni minimum. Puisqu'ils sont égaux, le test de vraisemblance est positif : c'est pour cela que l'affichage se termine par /MN, mais puisqu'il n'y a ni maximum ni minimum l'affichage commence par 00. Les deux signes + à gauche et à droite de 00/MN invitent l'utilisateur à accroître sur chacun des capteurs la pression qu'il exerce. L'afficheur indique donc +00/MN+

Lorsque l'appareil est appuyé sur l'artère radiale avec une force légère et à peu près constante. il y a détection d'un premier maximum. puis d'un deuxième maximum. calcul d'une première période T1. calcul d'un premier rapport R1. calcul d'un premier rapport M1.

L'apparail détecte ensuite un troisième maximum et calcule une deuxième période T2. un deuxième rapport R2 et un deuxième rapport M2.

L'appareil détecte ensuite un quatrième maximum et calcule une troisième période T3. un troisième rapport R3 et un troisième rapport M3. et ainsi de suite pendant N cycles cardiaques sans gêner en rien le passage du flux sanguin.

Dès la première mesure de T1. son inverse 1/T1 est calculé et ce résultat multiplié par 60 et arrondi à l'unité la plus proche est transmis pendant un temps égal à T1/2 à l'afficheur. C'est le rythme cardiaque. Lorsque plus de deux chiffres sont nécessaires. le signe / disparaît pour devenir le chiffre des unités.

Si l'appareil est placé bien perpendiculairement à l'artère. les deux signes + continuent à être affichés. Dans le cas contraire. l'un des + se transforme en -. invitant l'utilisateur à appuyer moins fort de ce côté de l'appareil.

Dès la deuxième mesure de T2, c'est T2 qui sert à calculer le nouveau rythme cardiaque transmis à l'afficheur pendant une demi-période.

A la troisième mesure, c'est T3 qui sert à calculer le nouveau rythme cardiaque transmis à l'afficheur pendant une demi-période.

Ainsi, pendant tout le temps que l'utilisateur exerce une force légère plus ou moins constante et qu'il observe le rythme cardiaque clignotant de son patient ou de lui-même, ce qui lui permet de compter le nombre de périodes et de détecter une éventuelle arythmie, il est sûr que les deux détecteurs de force de son appareil sont bien positionnés le long de l'artère radiale. Il sait que pendant tout ce temps les constantes R et M mises en mémoire continuent à être calculées et que leur moyenne sera de plus en plus proche du rapport $P_S/P_D$ des pressions systolique et diastolique, d'une part, et du rapport M de la pression moyenne $P_M$ à la pression diastolique $P_D$, d'autre part.

Il est en effet facile de constater que le rapport R est identique au rapport existant entre les pressions systolique et diastolique tant que la force de compression appliquée sur la paroi extérieure de l'artère est inférieure à la pression diastolique.

Au bout d'une dizaine de cycles cardiaques, l'utilisateur augmente aussi lentement que possible la force qu'il exerce sur l'artère radiale et, pour une certaine valeur F

0136212

15

de cette force, la circulation est perturbée sous l'un ou sous l'autre des dynamomètres, ou sous les deux dynamomètres à la fois mais de façons différentes, ce qui rend différentes les formes des signaux fournis par les dynamomètres et ce qui rend négatif le test de vraisemblance. Il est en effet connu que, lorsque la force de compression appliquée sur la paroi extérieure de l'artère augmente jusqu'à être égale à l'effet de la pression diastolique sur la paroi intérieure de l'artère, le débit sanguin commence à être perturbé. C'est la détection de cette perturbation qui est la raison d'être du second dynamomètre disposé en série avec le premier dynamomètre, mais il est évident qu'un seul dynamomètre suffirait à l'analyse des forces en présence si l'utilisateur était capable d'exercer une force très lentement et régulièrement croissante après avoir placé son appareil exactement sur l'axe de l'artère radiale. On comprend également l'aide apportée par la présence d'un second dynamomètre, cet agencement permettant, par comparaison des signaux fournis par les deux dynamomètres, de déceler le bon ou le mauvais positionnement de l'appareil sur l'artère radiale, cette aide étant particulièrement appréciable pour un utilisateur ne possédant aucune compétence spéciale dans ce domaine. Donc, pour une certaine valeur de la force exercée sur l'artère radiale par l'utilisateur de l'appareil, au Nième cycle cardiaque, le test de vraisemblance disparaît, ce qui déclenche les opérations suivantes :

1°) la moyenne arithmétique des forces mesurées par les dynamomètres à cet instant est mise en mémoire comme étant la pression diastolique $P_D$ ;

2°) la pression systolique $P_S$ est calculée en multipliant $P_D$ par la moyenne des R mis en mémoire ;

3°) la pression moyenne $P_M$ est calculée en multipliant $P_D$ par la moyenne des M mis en mémoire ;

4°) l'afficheur n'affiche plus le rythme cardiaque mais les pressions systolique et diastolique ; on lit par exemple 13/08 si la pression systolique est de 130 mm de mercure et si la pression diastolique est de 80 mm de mercure, unité de pression couramment utilisée dans le milieu médical.

Après avoir pris connaissance de $P_S$ et de $P_D$, l'utilisateur éloigne l'appareil de l'artère radiale, et le test de vraisemblance, qui réapparaît puisque les signaux des deux dynamomètres redeviennent égaux, fait adresser à l'afficheur non plus les valeurs de $P_S$ et de $P_D$ mais celles de $P_M$. On peut lire alors, par exemple MO9,3 si la pression artérielle moyenne est de 98 mm de mercure, et ceci jusqu'à ce que l'appareil soit mis hors tension.

Il est évident pour l'homme de l'art que les dynamomètres utilisés fourniront de préférence des informations digitales et que celles-ci seront avantageusement traitées par des circuits à semi-conducteur du type micro-processeur d'un usage courant aujourd'hui.

Il est également évident pour l'homme de l'art que l'appareil objet de la présente invention peut recevoir de nombreuses modifications tant dans le nombre des dynamomètres que dans leur type et que dans la façon de traiter ~t de présenter leurs signaux. que dans la présentation de l'appareil dont les afficheurs 17. 17´ et 17" ou le corps 16 peuvent être déportés au bout d'un cordon, être reliés à l'extrémité 18 par un dispositif élastique ou articulé. émettre à distance la mesure ou être remplacés par un dispositif vocal. ou effectuer la moyenne des mesures effectuées au cours d'une période de temps donnée. par exemple une journée. afin de surveiller les malades hypertendus et ajuster leur traitement anti-hypertensif. sans sortir du cadre de l'invention.

Par exemple l'appareil de l'invention peut être adapté pour la mise en oeuvre d'une méthode de mesure dite méthode oscillométrique. A cet effet l'appareil est agencé comme représenté à la figure 7 sur laquelle les éléments identiques à ceux de la figure 6 sont désignés à l'aide des mêmes références numériques.

Le bloc 43 soustrait à chaque cycle le dernier MIN du dernier MAX et donne donc l'amplitude de la pulsation.

Le bloc 44 calcule la pente de la variation de cette amplitude et contrôle les blocs 45. 46 et 47. qui mémorisent les forces correspondant respectivement à la pression systo-

18

lique $P_S$. à la pression diastolique $P_D$. et à la pression moyenne $P_M$.

En conséquence. l'invention ne doit pas être interprétée comme étant limitée au mode de réalisation particulier ici décrit. elle en englobe au contraire toutes les variantes.

REVENDICATIONS

1 - Appareil pour mesurer par voie externe la pression artérielle d'un patient, caractérisé en ce qu'il comprend :

- au moins un capteur de force (19) destiné, en fonctionnement, à être tenu en appui dans la gouttière du pouls avec une force sensiblement constante inférieure à celle créée par la pression diastolique du flux sanguin de l'artère radiale,

- des moyens de détection (21, 25, 27, 30) connectés au susdit capteur de force et agencés pour détecter les maxima et les minima des signaux de sortie du capteur de force,

- des premiers moyens de mémorisation (28, 31) connectés auxdits moyens de détection et agencés pour mémoriser lesdits maxima et lesdits minima,

- des premiers moyens de calcul (29, 32) connectés auxdits premiers moyens de mémorisation et agencés pour calculer respectivement la somme des maxima du signal de pression et la somme des minima du signal de pression,

- des deuxièmes moyens de calcul (33) connectés aux premiers moyens de calcul et agencés pour calculer la valeur moyenne $R_m$ du rapport de la somme des maxima du signal de pression à la somme des minima du signal de pression,

- des deuxièmes moyens de mémorisation (26) connectés au capteur de force et agencés pour mémoriser la valeur de la force d'appui correspondant à l'amorce de la perturbation du flux sanguin, cette force étant considérée comme la pression diastolique $P_D$,

- des troisièmes moyens de calcul (34) connectés aux deuxièmes moyens de mémorisation et aux deuxièmes moyens de calcul et agencés pour calculer la valeur $P_S$ du produit de la susdite valeur $P_D$ par la susdite valeur $R_m$,

- et des moyens d'affichage (17) connectés auxdits troisièmes moyens de calcul et aux deuxièmes moyens de mémorisation et agencés pour afficher, simultanément et dans

20

l'ordre, les valeurs $P_S$ et $P_D$.

2 - Appareil selon la revendication 1, caractérisé en ce qu'il comporte en outre :

- des quatrièmes moyens de calcul (35, 36) connectés aux moyens de détection et agencés pour calculer la valeur intégrale du signal au cours d'un cycle,
- des cinquièmes moyens de calcul (37) connectés aux quatrièmes moyens de calcul et aux moyens de détection des minima (30) et agencés pour effectuer le quotient M de la susdite valeur intégrale par la durée du cycle (temps séparant deux maxima consécutifs) et par le minimum du cycle considéré,
- des troisièmes moyens de mémorisation (38) connectés aux cinquièmes moyens de calcul et agencés pour mémoriser les rapports M calculés par lesdits cinquièmes moyens de calcul,
- des sixièmes moyens de calcul (39) connectés aux troisièmes moyens de mémorisation et agencés pour calculer la valeur moyenne des rapports M mémorisés dans lesdits troisièmes moyens de mémorisation,
- des septièmes moyens de calcul (40) connectés aux deuxièmes moyens de mémorisation et aux sixièmes moyens de calcul et agencés pour calculer le produit, considéré comme étant la valeur de la pression moyenne $P_M$, de la valeur de la pression diastolique $P_D$ par la valeur de la moyenne des rapports M, lesdits septièmes moyens de calcul ayant leur sortie connectée auxdits moyens d'affichage (17) pour l'affichage de la pression moyenne $P_M$.

3 - Appareil selon la revendication 1 ou 2, caractérisé en ce qu'il comprend en outre :

- des huitièmes moyens de calcul (41) connectés aux quatrièmes moyens de calcul (35) et agencés pour calculer l'inverse de la valeur de la période T fournie par les quatrièmes moyens de calcul et le multiplier par 60, ces huitièmes moyens de calcul ayant leur sortie connectée aux moyens d'affichage (17) en vue de l'affichage, au cours de chaque cycle cardiaque, du rythme cardiaque.

4 - Appareil selon l'une quelconque des revendications 1 à 3, caractérisé en ce qu'il comporte en outre des moyens d'analyse (24) de la forme de la variation instantanée de la pression artérielle, ces moyens d'analyse (24) ayant leur sortie connectée à une entrée de commande des seconds moyens de mémorisation (26) de manière à commander la mise en mémoire de la force F exercée par le capteur de force (19).

5 - Appareil selon la revendication 4, caractérisé en ce que les moyens d'analyse (24) comprennent au moins un second capteur de force (20), les deux capteurs (19, 20) étant destinés, dans la position de fonctionnement de l'appareil, à être disposés l'un à la suite de l'autre dans la gouttière du pouls le long de l'artère radiale, et en ce que sont en outre prévus :
- deux groupes de moyens de détection (21, 22, 23) connectés respectivement aux deux capteurs, et
- des moyens comparateurs (24) dont deux entrées sont connectées respectivement aux deux groupes de moyens de détection et dont la sortie est connectée à des moyens de signalisation (17') et (17") du positionnement de l'appareil.

6 - Appareil selon la revendication 5, caractérisé en ce qu'il comporte en outre des moyens sélecteurs (42) connectés à la sortie des moyens comparateurs (24) et aptes à connecter les moyens d'affichage (17) sélectivement avec les sorties des huitièmes moyens de calcul (41) lorsque les deux capteurs fournissent deux signaux sensiblement identiques correspondant à des forces d'appui respectives de ces deux capteurs inférieures à la force créée par la pression diastolique, puis avec les sorties des troisièmes moyens de calcul (34) et des deuxièmes moyens de mémorisation (26) lorsque les deux capteurs fournissent deux signaux sensiblement différents correspondant à des forces d'appui respectives de ces deux capteurs au moins égales à la force créée par la pression diastolique, puis enfin avec les huitièmes moyens de calcul (41) lorsque les deux capteurs fournissent des signaux à nouveau sensiblement identiques correspondant à des forces d'appui respectives des deux capteurs à nouveau inférieures à la force créée par la pression diastolique. ce grâce à quoi. par modification de la force d'appui de l'appareil sur la gouttière du pouls. les moyens d'affichage produisent successivement l'indication de la valeur du rythme cardiaque. puis des valeurs des pressions systolique $P_S$ et diastolique $P_D$. puis enfin de la valeur de la pression artérielle moyenne $P_M$.

7. Appareil selon l'une quelconque des revendications 1 à 6.caractérisé en ce qu'il comprend en outre des moyens soustracteurs (43) ayant des entrées connectées respectivement aux sorties des moyens de détection des maxima (27) et des minima (30) et agencés pour soustraire à chaque cycle le dernier MIN du dernier MAX et fournir un signal représentatif de l'amplitude de la pulsation. ainsi que des moyens de calcul supplémentaires (44) -aptes à calculer la pente de la variation de ladite amplitude- dont 1 entrée est connectée à la sortie desdits moyens soustracteurs (43) et dont les sorties sont raccordées à des moyens de mémorisation (45 à 47) mémorisant les forces correspondant respectivement à la pression systolique $P_S$. à la pression diastolique $P_D$ et à la pression moyenne $P_M$. ce grâce à quoi cet appareil est apte à mettre en oeuvre la méthode oscillométrique c'est-à-dire l'analyse de l'amplitude des oscillations au cours de l'annulation d'une force d'appui supérieure à celle exercée par la pression systolique du flux sanguin.

8. Appareil selon l'une quelconque des revendications 5 à 7, caractérisé en ce qu'il est muni d'un dispositif de suspension qui. combiné avec un dispositif de préhension. permet d'appuyer les deux capteurs avec deux forces égales progressivement croissantes ou progressivement décroissantes alors que l'utilisateur exerce une force constante ou nulle.

9. Appareil selon l'une quelconque des revendications 1 à 8, caractérisé en ce qu'il totalise et fournit la moyenne des mesures effectuées au cours d'une période de temps donnée.

10. Procédé pour mesurer par voie externe la pression artérielle d'un patient, caractérisé en ce qu'il comprend les étapes suivantes :

- on appuie au moins un capteur de force dans la gouttière du pouls avec une force sensiblement constante inférieure à celle créée par la pression diastolique du flux sanguin de l'artère radiale.

- on détecte les maxima et les minima des signaux fournis par le capteur et représentatifs de la forme de la pression artérielle, pendant une durée de temps correspondant à plusieurs cycles cardiaques

- on détermine, pour chaque cycle cardiaque, le rapport R du maximum du signal de pression au minimum du signal de pression mesurés au cours de chaque cycle cardiaque.

- on augmente progressivement la force d'appui du capteur jusqu'à perturber le flux sanguin, on détecte la déformation du signal de pression et on considère la force d'appui correspondant à l'amorce de la perturbation comme étant la pression diastolique $P_D$.

- on détermine, à la fin de la susdite durée de temps, la moyenne des rapports R précédemment calculés.

- on détermine le produit de la valeur de la pression diastolique $P_D$ par la moyenne des rapports R, ce produit étant considéré comme étant la valeur de la pression systolique $P_S$, et

- on affiche les valeurs $P_S$ et $P_D$.

11. Procédé selon la revendication 7, caractérisé en ce qu'en outre :

- on détermine, pour chaque cycle cardiaque, le rapport M de la pression moyenne, intégrale du signal fourni par le capteur pour ledit cycle divisé par la durée du cycle, au minimum mesuré au cours du cycle considéré.

- on détermine. à la fin de la susdite durée de temps. la valeur moyenne des rapports M;

- on détermine alors le produit de la valeur de la pression diastolique $P_D$ par la moyenne des rapports M. ce produit étant considéré comme étant la valeur de la pression artérielle moyenne $P_M$. et

- on affiche la valeur $P_M$.

12. Procédé selon la revendication 7 ou 8. caractérisé en ce qu'entre le début et la fin de la susdite durée de temps.

- on détecte la période de chaque cycle cardiaque.

- on détermine l'inverse de cette période du cycle cardiaque et on multiplie par 60 cette valeur inverse. et

- on affiche la valeur du rythme cardiaque.

13. Procédé selon l'une quelconque des revendications 7 à 9. caractérisé en ce qu'on utilise au moins deux capteurs de force disposés l'un à la suite de l'autre dans la gouttière du pouls le long de l'artère radiale. et en ce qu'on compare les signaux fournis par ces deux capteurs pour déterminer que l'appareil est bien positionné et que la force d'appui est inférieure ou respectivement au moins égale à la force créée par la pression diastolique du flux sanguin de l'artère radiale selon que les signaux fournis sont identiques ou respectivement différents.

9. Appareil selon l'une quelconque des revendications 1 à 8, caractérisé en ce qu'il totalise et fournit la moyenne des mesures effectuées au cours d'une période de temps donnée.

10. Procédé pour mesurer par voie externe la pression artérielle d'un patient. caractérisé en ce qu'il comprend les étapes suivantes :

- on appuie au moins un capteur de force dans la gouttière du pouls avec une force sensiblement constante inférieure à celle créée par la pression diastolique du flux sanguin de l'artère radiale.

- on détecte les maxima et les minima des signaux fournis par le capteur et représentatifs de la forme de la pression artérielle. pendant une durée de temps correspondant à plusieurs cycles cardiaques

- on détermine. pour chaque cycle cardiaque. le rapport R du maximum du signal de pression au minimum du signal de pression mesurés au cours de chaque cycle cardiaque.

- on augmente progressivement la force d'appui du capteur jusqu'à perturber le flux sanguin. on détecte la déformation du signal de pression et on considère la force d appui correspondant à l'amorce de la perturbation comme étant la pression diastolique $P_D$.

- on détermine. à la fin de la susdite durée de temps. la moyenne des rapports R précédemment calculés.

- on détermine le produit de la valeur de la pression diastolique $P_D$ par la moyenne des rapports R. ce produit étant considéré comme étant la valeur de la pression systolique $P_S$. et

- on affiche les valeurs $P_S$ et $P_D$.

11. Procédé selon la revendication 7. caractérisé en ce qu'en outre :

- on détermine. pour chaque cycle cardiaque. le rapport M de la pression moyenne. intégrale du signal fourni par le capteur pour ledit cycle divisé par la durée du cycle, au minimum mesuré au cours du cycle considéré.

- on détermine. à la fin de la susdite durée de temps. la valeur moyenne des rapports M;

- on détermine alors le produit de la valeur de la pression diastolique $P_D$ par la moyenne des rapports M. ce produit étant considéré comme étant la valeur de la pression artérielle moyenne $P_M$. et

- on affiche la valeur $P_M$.

12. Procédé selon la revendication 7 ou 8. caractérisé en ce qu'entre le début et la fin de la susdite durée de temps.

- on détecte la période de chaque cycle cardiaque.

- on détermine l'inverse de cette période du cycle cardiaque et on multiplie par 60 cette valeur inverse. et

- on affiche la valeur du rythme cardiaque.

13. Procédé selon l'une quelconque des revendications 7 à 9. caractérisé en ce qu'on utilise au moins deux capteurs de force disposés l'un à la suite de l'autre dans la gouttière du pouls le long de l'artère radiale. et en ce qu'on compare les signaux fournis par ces deux capteurs pour déterminer que 1 appareil est bien positionné et que la force d'appui est inférieure ou respectivement au moins égale à la force créée par la pression diastolique du flux sanguin de l'artère radiale selon que les signaux fournis sont identiques ou respectivement différents.

Fig.1

Fig.2

Fig. 3

Fig. 4

Fig. 5

Fig. 6

Pl. III / 4

0136212

Fig. 7

**RAPPORT DE RECHERCHE EUROPEENNE**

Office européen
des brevets

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 880 145 (E.F. BLICK) <br><br> * En entier * | 1,4,5, 10,13 | A 61 B 5/02 |
| A | US-A-4 245 648 (G.A. TRIMMER) <br><br> * Colonne 2, ligne 39 - colonne 8, ligne 3; figures 1-7 * | 1,5,6, 10,13 | |
| A | FR-A-1 586 707 (COMPAGNIE FRANCAISE THOMSON HOUSTON-HOTCHKISS BRANDT) <br> * En entier * | 2,11 | |
| A | US-A-4 331 154 (R.L. BROADWATER) <br> * Colonne 4, ligne 3 - colonne 9, ligne 27; figures 1-5 * | 1,3,10 ,12 | |
| A | IEEE TRANSACTIONS ON BIO-MEDICAL ELECTRONICS, vol. BME-10, no. 2, avril 1963, pages 73-83; G.L. PRESSMAN et al.: "A transducer for the continuous external measurement of arterial blood pressure" <br> * En entier * <br><br> ---     -/- | 1 | DOMAINES TECHNIQUES RECHERCHES (Int. Cl.4) <br><br> A 61 B |

Le présent rapport de recherche a été établi pour toutes les revendications

| Lieu de la recherche <br> LA HAYE | Date d'achèvement de la recherche <br> 14-11-1984 | Examinateur <br> ZILLIOX J.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82

**Office européen des brevets**

**RAPPORT DE RECHERCHE EUROPEENNE**

| | DOCUMENTS CONSIDERES COMME PERTINENTS | | Page 2 |
|---|---|---|---|
| Catégorie | Citation du document avec indication, en cas de besoin, des parties pertinentes | Revendication concernée | CLASSEMENT DE LA DEMANDE (Int. Cl.4) |
| A | EP-A-0 041 222 (J. & P. RIECKMANN et al.) <br> * Page 8, ligne 3 - page 10, ligne 2; page 11, ligne 4 - page 14, ligne 12; figures 1-3 * | 1,10 | |
| | --- | | |
| A | US-A-3 926 179 (J.C. PETZKE et al.) <br> * Colonne 2, ligne 57 - colonne 15, ligne 23; figures 1-12 * | 1,10 | |
| | --- | | |
| A | US-A-3 769 964 (R.W. SMITH) <br> * Colonne 5, ligne 29 - colonne 9, ligne 53; figures 1-6, 23 * | 1 | |
| | --- | | |
| P,A | GB-A-2 118 719 (R.L. BROADWATER) <br> * En entier * | 7,12 | DOMAINES TECHNIQUES RECHERCHES (Int. Cl.4) |
| | ----- | | |

Le présent rapport de recherche a été établi pour toutes les revendications

| Lieu de la recherche <br> LA HAYE | Date d'achèvement de la recherche <br> 14-11-1984 | Examinateur <br> ZILLIOX J.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

OEB Form 1503 03.82